# EUROPEAN PATENT SPECIFICATION

(11) **EP 0 651 659 B1**
(45) Date of publication and mention of the grant of the patent: **09.04.1997**
(21) Application number: 93915861.4
(22) Date of filing: 09.07.1993
(51) Int. Cl.: A61L 25/00, A61K 35/54

(54) **A TWO COMPONENT FIBRIN-GLUE COMPOSITION FOR IMPROVING IN VITRO FERTILIZATION**
Zweikomponenten-Fibrinkleber zur Verbesserung von in-Vitro Befruchtung
COMPOSITION DE COLLE FIBRINE A DEUX COMPOSANTS SERVANT A AMELIORER LA FERTILISATION IN VITRO

(30) Priority: 18.07.1992 EP 92112295; 30.03.1993 EP 93105298
(43) Date of publication of application: 10.05.1995
(73) Proprietor: OMRIX BIOPHARMACEUTICALS S.A., 1170 Bruxelles (BE)
(72) Inventor: BARNEA, Efrach, 51 200 Tel Aviv (IL)
(74) Representative: Meyers, Hans-Wilhelm, Dr.Dipl.-Chem.
(86) International application number: EP9301797
(87) International publication number: WO9402182

(56) References cited:
- EP-A- 0 339 607
- WO-A-92/15341
- WO-A-92/22312
- GB-A- 2 102 811

## Description

This invention relates to an iso-osmolar two component fibrin-glue composition comprising the components A and B for improving in vitro fertilization.

In 1987 the first in vitro fertilization (IVF) baby was born in England. Thereafter, the in vitro fertilization became widely used throughout the world. Indications for in vitro fertilization include virtually any form of infertility.

Since the introduction of IVF the techniques have improved in all aspects of this method, for example, induction of ovulation, ovum recovery and so on except in one field where are still severe problems and complications which is the field of implantation of the embryo into the uterus. The incidence of implantation of the embryo inside the uterus is still 8 to 9 % per embryo or 20 to 30 % rate of pregnencies (since 3 to 4 embryos are normally transferred in each in vitro fertilization). It can be assumed that the transfer of young embryos (2 to 8 cells) to the uterus may play an important role in this failure to get higher rate of implantations. These embryos are not mature enough for implantation at this stage. On the other hand culturing the embryos outside the uterus for longer time is damaging the embryos and only about 30 % of them will develop to the blastocyte stadium which is the developmental stage at which normal implantation occurs. Attempts for improvement of the implantation using a fibrin-glue known in the art failed. The idea was to attach the embryo with this fibrin-glue on the walls of the uterus (endometrium).

EP 0 339 607 discloses a composition for the regeneration of articular cartilage and bones by implantation comprising cells expressing a chondrocyte phenotype, namely bone marrow derived chondrocytes or osteoblasts of autologous or homologous origin or homologous committed chondrocytes or autologous or homologous muscle fibroblast derived chondroytes or any other progenital cells from mesenchymal origin. Such cells are embedded in a biodegradable, biocompatible, biological resorbable immobilization vehicle consisting of fibrinogen, thrombin, CaCl₂, protease inhibitor and at least 10%, preferably 15 - 30% serum.

GB 2,102,811 discloses a tissue adhesive and method of producing the same. This tissue is based on human or animal proteins containing factor XIII, fibrinogen and an antibiotic. In order to achieve a high straining capacity of the adherences and the retarded antimicrobial efficacy, the ratio of factor XIII to fibrinogen expressed in units of fact XIII per gram of fibrinogen amounts to at least 500. There are mentioned various antibiotics which must be used in order to invoid infections.

WO 92/22312 discloses a tissue treatment composition especially an adhesive composition comprising
(i) fibrin or fibrinogen and
(ii) a biodegradable and biocompatible polymer capable of forming a viscous aqueous solution. In addition to gluing the tissue adhesive composition may be used for slow-release of a drug incorporated into it or for anti-adherence purposes for wound healing etc.

It is an object of this invention to provide a composition and a method for improving in vitro fertilization.

According to the invention the above mentioned problem can be solved with an iso-osmolar two component fibrin-glue composition comprising components A and B wherein
- component A comprises cryoprecipitate of whole blood containing fibrinogen and a protease inhibitor,
- component B comprises a proteolytic enzyme being capable of cleaving specifically fibrinogen and causing formation of the fibrin polymer and
- additionally ingredients for culturing embryonic cells of mamals in one or both of the components A and B.

The two component fibrin-glue composition of the invention is iso-osmolar. Therefore, the disadvantage of the glues of the prior art being hyper-osmolar is prevented. Hyper-osmolarity causes the death of the embryo by drying out phenomena.

If cryoprecipitate of whole blood or plasma is used as fibrinogen containing fraction of component A the sucess of in vitro fertilization is increased. Although, commercially available cryoprecipitate which was derived from whole blood can be used. It can be advantageous to concentrate the cryoprecipitate between a factor 2 and 5.

Preferably, the cryoprecipitate is virus inactivated. A procedure for virus inactivation is, for example, described in PCT/EP 91/00503. The basic principle of this method is the treatment of the cryoprecipitate with special detergents and removing the detergent later on from the cryoprecipitate. Preferably, the protease inhibitor present in component A is aprotinin and present in concentrations up to 10,000 U/ml based on total volume of component A. Aprotinin is commercially available under the trademark Trasylol^{R} or Antagosan^{R}. Also tranexamic acid [4-(aminomethyl)cyclohexane carboxylic acid] or its acceptable salts is a suitable agent which can be used instead of aprotinin or in combination with.

The second component B of the two component fibrin-glue composition of the invention is prepared by solution of proteolytic enzyme being capable of cleaving specifically fibrinogen. Preferably, thrombin has been used which was isolated from plasma of human beings or mamals such as bovine. The thrombin can be delivered in a lyophilized form. The reconstitution of thrombin occurs with a solution containing calcium chloride. The concentration of the protease specifically for the cleavage of fibrinogen depends on the method of transferring the embryo into the uterus. Basically, if a slow working two component fibrin-glue composition is desired the concentration of thrombin should be lower whereas if a fast working fibrin glue is desirable the concentration of thrombin should be higher (fast fibrin-glue). Typically the concentration range of thrombin is 0.4 to 4 units/ml in a slow working fibrin-glue.

Another preferred embodiment of the fibrin-glue of the present invention comprises as component B a proteolytic enzyme which is isolated from snake venom. The snake venom enzyme batroxobin which can be isolated from the south american pit viper Bothrpos moujini can be used. Chemically this venom is a single chain glyco peptide with a molecular weight of approximately 36,000. It is known under the name Defibrase^{R} which causes cleavage of the ala-16-arg/17 glue bond in fibrinogen which causes the release of fibrino peptide A and the formation of monomeric fibrin I.

The use of any venom converting fibrinogen to fibrin such as Defibrase or Reptilase is preferred when the cryo-precipitate is made from autologous source and the use of the venom will avoid the use of human blood product.

According to the invention there must be present ingredients for culturing embryonic cells of mamals in the fibrin-glue of the invention. This can be achieved either by admixing the ingredients into one or both of the components A and B of the invention or dissolve these ingredients in a liquid and mixing this liquid to one or both of components A and B. Preferred ingredients for culturing embryonic cells of mamals are those comprising calcium chloride, potassium chloride, magnesium sulfate, sodium chloride, sodium bicarbonate, sodium dihydrogenphosphate, D-glucose as well as phenol. Preferably, the solution has a pH of about 7.2. It can be advantageous to add additionally pyruvic acid and antibiotics, e. g. gentamycin.

A typical procedure for preparing component A in a virus free preparation is disclosed in PCT/EP 91/01850.

In a typical procedure the cryoprecipitate and the thrombin is dissolved in the medium for culturing the embryo. Of course, both components are separated in order to prevent starting of the clot reaction. The composition of two preferred culture mediums are given below. The first one is the so-called EBSS which is commercially available from Gibco Ltd. EBSS is the abbreviation for Earle's Balanced Salt Solutions. It can be purchased in liquid or powder form. The liquid contains anhydrous calcium chloride in amounts of 0.2 g/l, potassium chloride 0.4 g/l, MgSO₄ x 7 H₂O 0.2 g/l, sodium chloride 6.8 g/l, sodium bicarbonate 2.2 g/l, NaH₂PO₄ x 2H₂O 0.158 g/l and additionally D-glucose 1.0 g/l and phenol red 0.01 g/l. The respective powder form contains after being dissolved in one liter 0.20 g/L calcium chloride anhydrous, 0.4 g/l sodium chloride, 0.0977 g/l magnesium sulfate anhydrous, sodium chloride 6.80 g/l, NaH₂PO₄ x H₂O and the same D-glucose and phenol red content as in the liquid form. It can be advantageous to add pyruvic acid preferably in amounts of 0.015 g/l and gentamycin in amounts of 50 mg/l.

Depending on the field where the in vitro fertilization is carried out the medium can be modified in order to meet the special requirements which may be differ, for example, with respect with agricultural applications such as breeding. The above mentioned culture medium can be used also in human in vitro fertilization.

The advantage of the present fibrin-glue over the known fibrin-glue products having failed to improve the outcome of in vitro fertilization may be due to its increased anti-fibrinolytic activity preventing or postponing the embryo from one hand in the uterus on the other hand to degredate the clot. The two component fibrin-glue composition of the present invention enables the embryo to survive in the environment of the uterus before it is lodged and permenently fixed to the uterus wall. The surviving is supported by the medium containing ingredients for embryo cell culturing. Surprisingly, the high amounts of protease inhibitor like aprotinin do not interfere with the embryo but prevent the digest of the glue. Moreover, in another preferred embodiment of the present invention.

The advantageous features of the fibrin-glue of the invention are the presence of salts and nutrients which are important for in vitro growing of the embryo, it contains anti-fibrinolytic activity to postpone the early degradation of the glue either by embryo or by the uterus. Moreover, the antifibrinolytic potency of the embryo has to be balanced to the extent that it will enable the fetus to lyse a certain area around itself creating a halo like shape of fluid surrounded by the fibrin-glue.
Under certain circumstances it could be recommendable to include a growth factor that will enhance and speed up the maturation of the fetus.

The use of cryoprecipitate as a source of fibrinogen rather than a fibrinogen concentrate is advantageous since the former one contains ingredients like fibronectin, von Willebrand factor etc. which may be important for adhesion. Moreover, a cryoprecipitate may also contain other agents such as proteins or low molecular substances which support the development and lodging of the embryo. Also compounds having wound healing properties such as hyaluronic acid can be used.

For preparing the two component fibrin-glue composition of the invention (as it can be used in the experiments as described above) cryoprecipitate is dissolved in culture medium as described above. Then aprotinin or tranexamic acid is added to the cryoprecipitate. The concentration of aprotinin in the cryoprecipitate containing component A is preferably in the range of 6,000 to 10,000 u/ml. The concentration of tranexamic acid is preferably 10 - 200 µg/ml (final concentration). This is equivalent to an aprotinin activity of about 3,000 to 10,000 KIU/ml. The final concentration of aprotinin in the mixture of component A and B is lower because of the dilution when component B is added. The dilution factor depends on the amount of component B which is added. The concentration of fibrinogen in the cryoprecipitate solution is relatively low compared with other fibrin-glues since there is no need for strong tensile strength. Preferred are concentrations of fibrinogen in the range of 5 to 20 g/l.

The embryo is injected for example via the following method. The embryo is placed in the reconstituted cryoprecipitate solution in a culture dish. Then thrombin is added in concentrations of 0.4 to 4 units. The thrombin concentration determines the clotting time of the glue. the embryo is sucked in the culture fluid and injected into the uterus.

## Claims

1. An iso-osmolar two component fibrin-glue composition comprising the components A and B wherein
- component A comprises fibrinogen and a protease inhibitor,
- component B comprises a proteolytic enzyme being capable of cleaving specifically fibrinogen and causing formation of the fibrin polymer and
- additionally ingredients for culturing embryonic cells of mamals in one or both of the components A and B.

2. The composition of claim 1 wherein the fibrinogen containing a fraction of component A is cryoprecipitate of whole blood.

3. The composition of claims 1 and/or 2 wherein the protease inhibitor of component A is aprotinin in amounts up to 10,000 u/ml based on total volume of component A and/or 4-(aminomethyl)cyclohexane carboxylic acid or its pharmaceutically acceptable salts.

4. The composition of any one of the claims 1 to 3 wherein the proteolytic enzyme of component B is thrombin or a proteolytic enzyme derived from snake venom such as batroxobin isolated from venom of the south american pit viper Bothrpos moujini.

5. The composition of any one of the claims 1 to 4 wherein component A is virus inactivated cryoprecipitate.

6. The composition of any one of the claims 1 to 5 wherein the culture medium for growing embryonic cells contains calcium chloride, potassium chloride, magnesium sulfate, sodium choride, sodium bicarbonate, sodium dihydrogenphosphate, D-glucose as well as phenol red at a pH of about 7.2 and containing optionally pyruvic acid and/or gentamycin.

7. The composition of any one of the claims 1 to 6 having an agent with wound healing such as hyaluronic acid.

8. Method of improving the success of in vitro fertilization in breeding of livestock or cattle breeding whereby the embryo is transferred together with the two component fibrin-glue composition of any one of the claims 1 to 6 into the uterus.

## Patentansprüche

1. Isoosmolare zweikomponentige Fibrinklebezusammensetzung, umfassend die Komponenten A und B, wobei
- Komponente A Fibrinogen und einen Protease-Inhibitor umfaßt,
- Komponente B ein proteolytisches Enzym umfaßt, das Fibrinogen spezifisch zu spalten und die Bildung des Fibrinpolymers zu bewirken vermag, sowie
- zusätzlich Bestandteile zum Kultivieren embryonischer Zellen von Säugern in einem oder zwei der Komponenten A und B.

2. Zusammensetzung gemäß Anspruch 1, wobei es sich bei dem fibrinogenhaltigen Anteil von Komponente A um Kryopräzipitat von Vollblut handelt.

3. Zusammensetzung gemäß Anspruch 1 und/oder 2, wobei es sich bei dem Protease-Inhibitor von Komponente A um Aprotinin in Mengen von bis zu 10 000 E/ml, bezogen auf das Gesamtvolumen von Komponente A, und/oder um 4-(Aminomethyl)cyclohexancarbonsäure oder ihre pharmazeutisch annehmbaren Salze handelt.

4. Zusammensetzung gemäß einem der Ansprüche 1 bis 3, wobei es sich bei dem proteolytischen Enzym von Komponente B um Thrombin oder ein von einem Schlangengift abgeleitetes proteolytisches Enzym, wie Batroxobin, das aus dem Gift der südamerikanischen Grubenotter *Bothrops moujini* isoliert wird, handelt.

5. Zusammensetzung gemäß einem der Ansprüche 1 bis 4, wobei es sich bei Komponente A um virusinaktiviertes Kryopräzipitat handelt.

6. Zusammensetzung gemäß einem der Ansprüche 1 bis 5, wobei das Kulturmedium zum Kultivieren embryonischer Zellen Calciumchlorid, Kaliumchlorid, Magnesiumsulfat, Natriumchlorid, Natriumhydrogencarbonat, Natriumdihydrogenphosphat, D-Glucose sowie Phenolrot bei einem pH-Wert von etwa 7,2 enthält und gegebenenfalls Brenztraubensäure und/oder Gentamycin enthält.

7. Zusammensetzung gemäß einem der Ansprüche 1 bis 6, die ein Mittel zur Wundheilung, wie Hyaluronsäure, aufweist.

8. Verfahren zur Verbesserung des Erfolgs einer in-vitro-Fertilisation bei der Vieh- oder Rinderzucht, wobei der Embryo zusammen mit der zweikomponentigen Fibrinklebezusammensetzung gemäß einem der Ansprüche 1 bis 6 in den Uterus übertragen wird.

## Revendications

1. Une composition de colle fibrineuse iso-osmolaire à deux composants, comprenant les composants A et B dans lesquels
- le composant A comprend du fibrinogène et un inhibiteur de protéase,
- le composant B comprend une enzyme protéolytique capable de cliver spécifiquement le fibrinogène et de provoquer la formation du polymère de fibrine, et
- de plus, des ingrédients pour la culture de cellules embryonnaires de mammifères dans l'un des composants A et B ou les deux.

2. La composition de la revendication 1, dans laquelle la fraction contenant du fibrinogène du composant A est un cryoprécipité de sang entier.

3. La composition des revendications 1 et/ou 2, dans laquelle l'inhibiteur de protéase du composant A est l'aprotinine en des quantités d'au plus 10 000 U/ml par rapport au volume total du composant A et/ou l'acide 4-(aminométhyl)cyclohexane-carboxylique ou ses sels pharmaceutiquement acceptables.

4. La composition de l'une quelconque des revendications 1 à 3, dans laquelle l'enzyme protéolytique du composant B est la thrombine ou une enzyme protéolytique dérivée de venin de serpent telle que la batroxobine isolée du venin de trigonocéphale d'Amérique du Sud *Bothrops moujini*.

5. La composition de l'une quelconque des revendications 1 à 4, dans laquelle le composant A est un cryoprécipité ayant subi un traitement d'inactivation de virus.

6. La composition de l'une quelconque des revendications 1 à 5, dans laquelle le milieu de culture pour la croissance de cellules embryonnaires contient du chlorure de calcium, du chlorure de potassium, du sulfate de magnésium, du chlorure de sodium, du bicarbonate de sodium, du dihydrogénophosphate de sodium, du D-glucose ainsi que du rouge de phénol à un pH d'environ 7,2 et contient facultativement de l'acide pyruvique et/ou de la gentamycine.

7. La composition de l'une quelconque des revendications 1 à 6, contenant un agent ayant des propriétés cicatrisantes tel que l'acide hyaluronique.

8. Procédé pour améliorer le taux de réussite de la fécondation *in vitro* dans l'élevage de bétail ou l'élevage de bovins, selon lequel l'embryon est transféré dans l'utérus avec la composition de colle fibrineuse à deux composants de l'une quelconque des revendications 1 à 6.
